# EUROPEAN PATENT SPECIFICATION

(11) **EP 4 395 853 B1**
(45) Date of publication and mention of the grant of the patent: **07.10.2026**
(21) Application number: 22765555.2
(22) Date of filing: 18.08.2022
(51) Int. Cl.: A61M 5/32, A61M 5/20

(54) **ACTIVATION MEMBER AND SUBASSEMBLY FOR A MEDICAMENT DELIVERY DEVICE**
AKTIVIERUNGSELEMENT UND UNTERBAUGRUPPE FÜR EINE MEDIKAMENTENABGABEVORRICHTUNG
ÉLÉMENT D'ACTIVATION ET SOUS-ENSEMBLE POUR DISPOSITIF D'ADMINISTRATION DE MÉDICAMENTS

(30) Priority: 01.09.2021 EP 21194407
(43) Date of publication of application: 10.07.2024
(73) Proprietor: SHL Medical AG, 6302 Zug (CH)
(72) Inventor: CARLSSON, Daniel, 131 28 Nacka Strand (SE)
(86) International application number: PCT/EP2022/073051
(87) International publication number: WO 2023/030903

(56) References cited:
- WO-A1-2009/114542
- WO-A1-2016/202555
- WO-A1-2020/143991
- US-A1- 2005 101 919
- US-A1- 2013 317 427
- US-A1- 2018 272 075

## Description

### Technical field

The present invention relates to an activation member for a medicament delivery device which enables a two-step activation of the medicament delivery device. Further, the present invention relates to a subassembly for a medicament delivery device comprising said activation member. Further, the present invention relates to a medicament delivery device comprising said subassembly or said activation member, so that the medicament delivery device can be activated in two-steps, i.e. removing an end cap and displacing a needle cover.

### Related Art

Medicament delivery devices, especially disposable single-dose auto-injectors, are often intended to be used by users in a home environment, enabling the user to self-apply a medical substance, for example insulin or hormones, without a medically trained person being present. Resulting from that, easy and safe handling of such devices is a key design aspect. Thus, depending on the intended use and type of medicament, medicament delivery devices having a varying degree of automatic functions have been developed to facilitate injection of medicaments in a reliable and safe manner.

Auto-injectors with a penetration function usually comprise a housing, a container holder carrying a medicament container with a needle and a plunger rod as well as a compression spring providing a force to move the plunger rod with respect to the housing and/or container holder. Such auto-injectors often also comprise a needle cover and an end cap protecting the tip of the needle in order to prevent injuries and contamination of the needle, when the injector is not used and/or after the injection is completed. Moreover, existing auto-injectors usually comprise an activation button that enables the user to determine the time of injection.

When said auto-injectors are actuated, the container holder and the plunger rod, which are slidable in the housing, are jointly driven towards the user's skin by the force of the compression spring, which causes the needle to penetrate the user's skin. After having reached a predetermined position, the plunger rod is moved towards the user's skin, thereby acting on a stopper inside the medicament container such that the medicament is expelled through the needle.

Some auto-injectors may further have an automatic safety function for preventing accidental needle sticks by covering the needle with the needle cover when the injector is withdrawn from the user's skin.

Prior to the actuation, the auto-injector is in a secured state, in which the end cap is attached to the housing and a movement of the plunger rod is inhibited. Initiating an injection usually requires the user to activate the injector by consecutively performing three steps. First, the user needs to remove the end cap from the housing. After that, the user needs to press the needle cover against the intended injection site with a predetermined force. Third, the user needs to press the activation button which initiates the actuation.

Such a the three-step activation as used in conventional auto-injectors and provides sufficient safety when it comes to avoiding injuries and premature medicament discharge, for example when the injector is dropped to the floor. However, from a usability point of view, an activation requiring three user interactions is disadvantageous because it increases the risk of operating errors. In the worst case such errors might block the injector, so the intended injection cannot be performed.

One of the most common operating errors is that the activation steps are not performed in the correct order, for example the activation button is pressed before the needle cover is pressed against the user's skin. Despite that, even when performed in the correct order, pressing the activation button still might be impossible, for example when the force with which the needle cover is pressed against the user's skin is less than the predetermined threshold. In this scenario, the injection cannot be initiated, either.

It is an object of the present invention to simplify the activation process of a medicament delivery device, especially a disposable single-dose auto-injector, while still preventing injuries or accidental medicament discharge.

US 2013/317427 A1 discloses an auto-injector for administering a dose of a liquid medicament. US 2005/101919 A1 discloses an injection device. WO 2020/143991 A1 and WO 2016/202555 A1 disclose a medicament delivery device. US 2018/0272075 A1 disclose a safety assembly for a medicament delivery device. WO 2009/114542 A1 disclose a safety member for use with an injection device.

### Summary of the invention

The above-mentioned objects are achieved, and the problems are overcome by the medicament delivery device according to independent claim 1.

Further aspects, improvements and variations are disclosed in the dependent claims, the figures and the description.

In the present application, when the term "distal" is used, this refers to the direction pointing away from the injection site, i.e. the point at which the needle penetrates the user's body in order to inject the medicament. When the term "distal part/end" is used, this refers to the part/end of the delivery device, or the parts/ends of the members thereof, which is/are located furthest away from the injection site. Correspondingly, when the term "proximal" is used, this refers to the direction pointing to the injection site. When the term "proximal part/end" is used, this refers to the part/end of the delivery device, or the parts/ends of the members thereof, which is/are located closest to the injection site.

The activation member of the medicament delivery device, according to an embodiment of the present disclosure extends along a longitudinal axis from a proximal to a distal end of the medicament delivery device. The activation member comprises an outer part and an inner part coaxially connected to the outer part. The outer part comprises a needle cover contact surface configured to contact a needle cover of the medicament delivery device. The inner part comprises at least one flexible arm extending distally and having an inclined end surface and a plunger rod support at its distal end. The inclined end surface is radially inwardly inclined in the proximal direction. The activation member further comprises at least two spring supports each extending distally and being configured to support a distal end of a drive spring of the medicament delivery device. The plunger rod support is configured to engage with a plunger rod of the medicament delivery device in order to inhibit a proximal movement of the plunger rod. The inclined end surface is configured to interact with a distal end part of the medicament delivery device, preferably a distal end cap, causing a radial outward deflection of the at least one flexible arm thereby disengaging the plunger rod support from the plunger rod, when the activation member is distally displaced by the needle cover of the medicament delivery device.

Preferably, the proximal movement of the plunger rod is inhibited by the plunger rod support when the medicament delivery device is in a non-activated state, i.e. a state prior to the distal displacement of the activation member by the needle cover, which causes a radial outward deflection of the at least one flexible arm.

Preferably, a circumferential surface of the inner part is formed of the at least one flexible arm, the at least two spring supports, and at least one slot between each flexible arm and each spring support. The at least one slot is configured to kinematically decouple the at least one flexible arm from each of the at least two spring supports in order to enable a radial outward deflection of the at least one flexible arm.

Preferably, the inner part and the outer part of the activation member overlap along a part of their length. Further preferably, the outer part is connected to or integral with the inner part via at least one support extending radially between at least one of the springs supports and a connection structure of the outer part.

Preferably, each plunger rod support has a radially inwards extending protrusion configured to form a stop surface for engagement with a distal portion of the plunger rod, so that the plunger rod is engaged with the plunger rod support, when the medicament delivery device is in a non-activated state.

Preferably, each spring support comprises a protrusion or step extending radially inwards.

Further preferably, the protrusion or step have a proximally facing support surface configured to support the distal end of the drive spring.

Preferably, the support is located at the spring support radially opposite to the protrusion or step.

Preferably, when there are two spring supports, the two spring supports are positioned with an offset between 90° and 270° in a circumferential direction of the activation member. More preferably, the two spring supports arms are positioned with an offset of 180° in a circumferential direction of the activation member.

Preferably, there are more than two spring supports distributed evenly in a circumferential direction.

Preferably, the activation member further comprises a distal spring support surface configured to interact with a distal spring of the medicament delivery device. Preferably the distal spring support surface is formed by the support or a distal end surface of the connection structure.

Preferably, the at least one flexible arm and the at least two spring supports are alternately arranged in circumferential direction.

Preferably, the proximally facing support surface of the protrusion or step of the spring support is located more proximal compared to the distally facing stop surface of the radially inwards extending protrusion of the plunger rod support.

Preferably, the inner part comprises at least two flexible arms each extending distally and each having an inclined end surface and a plunger rod support at its distal end. More preferably, the inner part comprises at least three flexible arms each extending distally and each having an inclined end surface and a plunger rod support at its distal end.

Preferably, the at least two flexible arms and the at least two spring supports are alternately arranged in circumferential direction. More preferably, the angular distribution of the at least two flexible arms and the at least two spring supports is even in a circumferential direction.

Preferably, when there are two flexible arms, the two flexible arms are positioned with an offset between 90° and 270° in a circumferential direction of the activation member. More preferably, the two flexible arms are positioned with an offset of 180° in a circumferential direction of the activation member.

Preferably, when there are two flexible arms and two spring supports alternately arranged in a circumferential direction, the angle between one flexible arm and each of the spring supports and the angle between one spring support and each of the flexible arms is 90° in a circumferential direction.

Preferably, the inner part comprises at least three spring supports.

Preferably, the spring supports are distributed in a circumferential direction with respect to the number of flexible arms, such that tilting of the drive spring of the medicament delivery device can be prevented. More preferably, the angular distribution of the spring supports in a circumferential direction is even.

A subassembly of the a medicament delivery device comprises a housing and an activation member according to one or more of the foregoing paragraphs or embodiments, respectively. The activation member is coaxially arranged within the housing and distally movable in relation to the housing. The subassembly further comprises a plunger rod coaxially arranged within the activation member and releasably connected to the distal end of the activation member, preferably releasably connected to the plunger rod support of the activation member. The subassembly further comprises a drive spring coaxially arranged between the plunger rod and the inner part of the activation member, preferably between the plunger rod and the spring supports of the activation member. The distal end of the drive spring is supported by the spring supports of the activation member and the proximal end of the drive spring is supported by a proximal part of the plunger rod, for example a proximal ledge of the plunger rod. The subassembly further comprises a distal end cap connected to the distal housing and a distal spring arranged between the distal end cap and the activation member, which is configured to apply a biasing force on the activation member.

Preferably, the subassembly is a distal subassembly for a medicament delivery device, and so the housing may be named a distal housing.

Preferably, the distal end cap comprises a guide hole along the longitudinal axis of the medicament delivery device configured to axially guide the distal end of the plunger rod when the activation member is distally displaced.

The distal end cap comprises a displacement structure configured to interact with the inclined end surface of the at least one flexible arm of the activation member in order to radially deflect the at least one flexible arm such that the plunger rod support is disengaged from the plunger rod, when the activation member is distally displaced.

The displacement structure is rigid. Preferably, the displacement structure is radially inclined, preferably with the same angle of inclination as the inclined end surface of the at least one flexible arm.

The medicament delivery device according to an embodiment of the present disclosure comprises a distal subassembly and a proximal subassembly. The distal subassembly is a subassembly according to one or more of the foregoing paragraphs.

Preferably, the proximal subassembly comprises a proximal housing connectable to the distal housing, a rotator coaxially connected to a distal inner part of the proximal housing and movable in relation to the proximal housing, a displaceable needle cover partially arranged within the proximal housing, a medicament container holder connected through the needle cover and the rotator to the proximal housing and configured to receive a medicament container. The proximal subassembly further comprises a needle cover spring disposed between the needle cover and the medicament container holder and configured to apply a biasing force on the needle cover. The needle cover is axially movable inside at least the proximal housing such that a distal end of the needle cover contacts the contact surface of the activation member whereby the activation member is distally displaced. In other words, a distal displacement of the needle cover distally displaces the activation member due to a contact of the distal end of the needle cover to the contact surface of the activation member.

Preferably, the rotator is rotationally and/or axially movable in relation to the proximal housing.

Preferably, the distal end of the needle cover comprises at least one arm extending distally and contacting the contact surface of the activation member in order to distally displace the activation member.

Preferably, the medicament delivery device is a disposable single-dose auto-injector.

The activation member according to an embodiment of the present disclosure enables the activation of a medicament delivery device in two steps, i.e. removal of the end cap and compression of the needle cover against the injection site. This reduces the risk of operating errors and leads to higher degree of automation in the injection process. Consequently, the injection process is simplified and can be easily and safely performed by non-trained users.

By the provision of at least two flexible arms on the inner part of the activation member, a uniform distribution of forces in a circumferential direction can be achieved, which is beneficial for ensuring a correct function of the medicament delivery device.

By the provision of at least two spring supports on the inner part of the activation member, a uniform distribution of forces in a circumferential direction can be achieved, which is beneficial for preventing titling of the drive spring and ensuring a correct function of the medicament delivery device.

The subassembly according to an embodiment of the present disclosure provides similar advantages.

The medicament delivery device according to an embodiment of the present disclosure is advantageous over the known devices since it can be activated with two activation steps without compromising safety aspects, e.g. the prevention of unintended product discharge due to a drop of the device. The medicament delivery device does not require an activation button, so it has less movable components and mechanisms which might cause failure.

### Brief description of the drawings

The following Figures below disclose an embodiment of the invention for illustrational purposes only. In particular, the disclosure within the Figures is not meant to limit the range of protection of the invention. The embodiment shown may be modified in many ways within the scope of the claims.
- Fig. 1: shows a perspective view of an activation member according to a preferred embodiment of the invention;
- Fig. 2: shows a zoomed perspective view of the distal portion of an inner part of the activation member according to a preferred embodiment of the invention;
- Fig. 3A: shows a partially cutaway perspective view of a subassembly according to a preferred embodiment of the invention;
- Fig. 3B: shows a different perspective of the subassembly of Fig. 3A;
- Fig. 4A: shows cross-sectional view of a distal end cap of the subassembly according to a preferred embodiment of the invention;
- Fig. 4B: shows cross-sectional view of the distal end cap of the subassembly in engagement with the distal end of the activation member according to a preferred embodiment of the invention;
- Fig. 5: shows a perspective view of a medicament delivery device according to a preferred embodiment of the invention; and
- Fig. 6: shows a perspective view of a proximal subassembly of the medicament delivery device of Fig. 5.

### Detailed description of the drawings

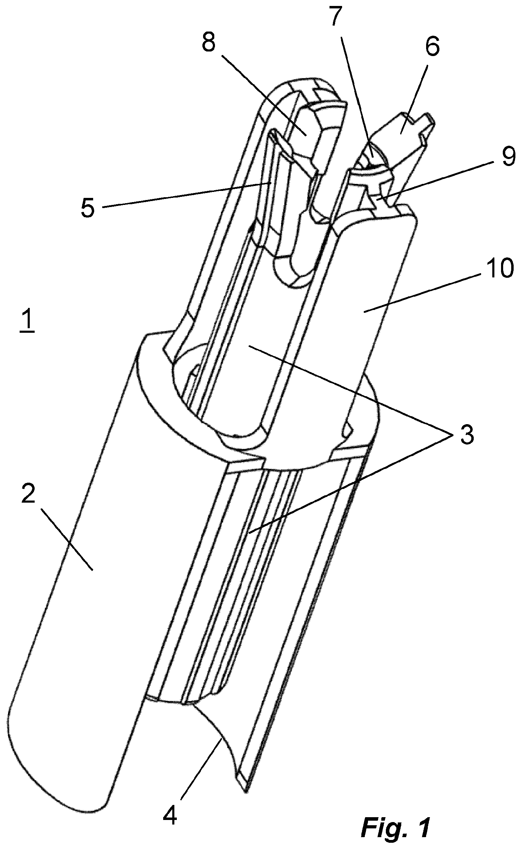

Fig. 1 shows a perspective view of an activation member 1 according to a preferred embodiment of the present disclosure. The activation member 1 comprises an outer part 2 and an inner part 3 coaxially connected to the outer part 2. The outer part 2 comprises a needle cover contact surface 4 configured to contact a needle cover of the medicament delivery device.

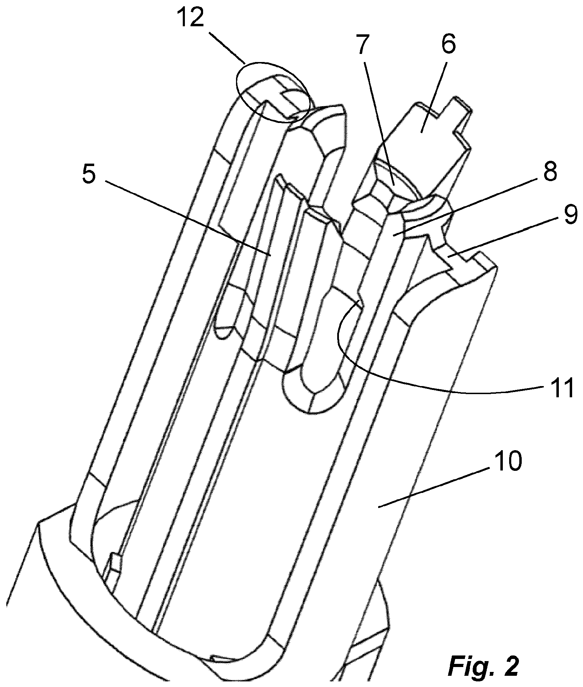

According to an embodiment, as shown in Fig. 2, the inner part 3 comprises two flexible arms 5 extending distally and having an inclined end surface 6 and a plunger rod support 7 at its distal end. The inclined end surface 6 is radially inwardly inclined in the proximal direction. As exemplarily shown, the plunger rod support 7 radially inwardly protrudes from the flexible arm 5.

According to an embodiment, the inner part 3 of the activation member 1 further comprises two spring supports 8 each extending distally. As shown, the spring supports 8 and the flexible arms 5 are distributed alternatingly along the circumference of the inner part 3, with a slot therebetween.

According to an embodiment, the outer part 2 is connected to or integral with the inner part 3 via two supports 9 extending radially between the spring supports 8 and a connection structure 10 of the outer part 2.

As further shown in Fig. 2, each spring support 8 comprises a protrusion or step extending radially inwards and having a proximally facing support surface 11 configured to support the distal end of a drive spring of the medicament delivery device. According to an embodiment, the support 9 is located radially opposite to the protrusion or step of the spring support 8. According to an embodiment, a distal spring support surface 12 is formed by the support 9 or a distal end surface of the connection structure 10.

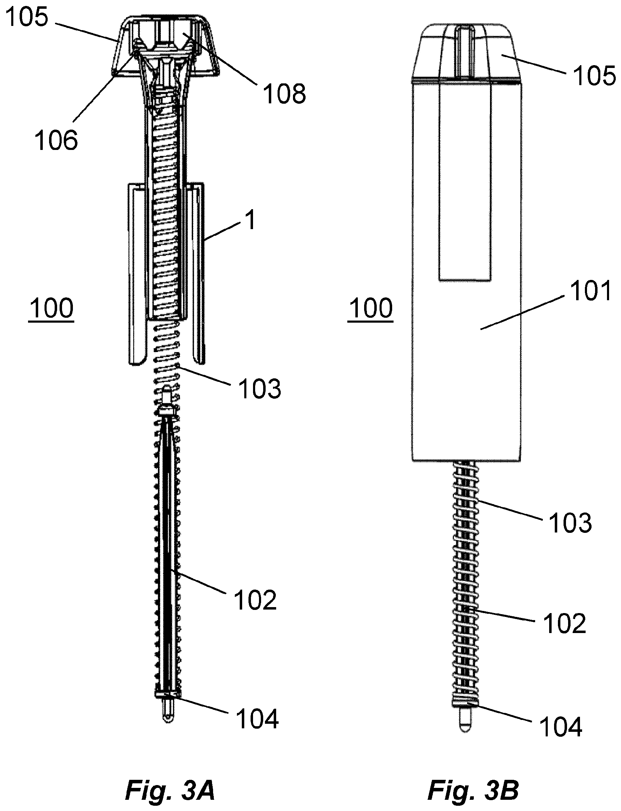

Figs. 3A and 3B show perspective views of a subassembly 100 according to a preferred embodiment of the present disclosure. The subassembly 100 comprises a housing 101 (not shown in the cross-sectional view of Fig. 3A) and an activation member 1 (not visible in Fig. 3B) according to one of the embodiments described in the foregoing paragraphs. The activation member 1 is coaxially arranged within the housing 101. The shown subassembly 100 further comprises a plunger rod 102 coaxially arranged within the activation member 1 and releasably connected to the plunger rod support 7 of the activation member 1. The shown subassembly 100 further comprises a drive spring 103 coaxially arranged between the plunger rod 102 and the spring supports 8 of the activation member 1, which is configured to proximally displace the plunger rod 102, when the plunger rod 102 is released from its engagement with the plunger rod support 7 of the activation member 1 due to a deflection of the flexible arms 5. In Fig. 3 the drive spring 103 is shown in an expanded state and so the plunger rod 102 is actually axially outside the activation member 1. However, in an assembled state the plunger rod 102 will be coaxially arranged within the activation member 1.

The distal end of the drive spring 103 is supported by the spring supports 8 of the activation member 1 and the proximal end of the drive spring 103 is supported by a proximal part of the plunger rod 102, for example a proximal ledge 104 of the plunger rod 102. The subassembly further comprises a distal end cap 105 connected to the housing 101 and a distal spring 106 arranged between the distal end cap 105 and the activation member 1.

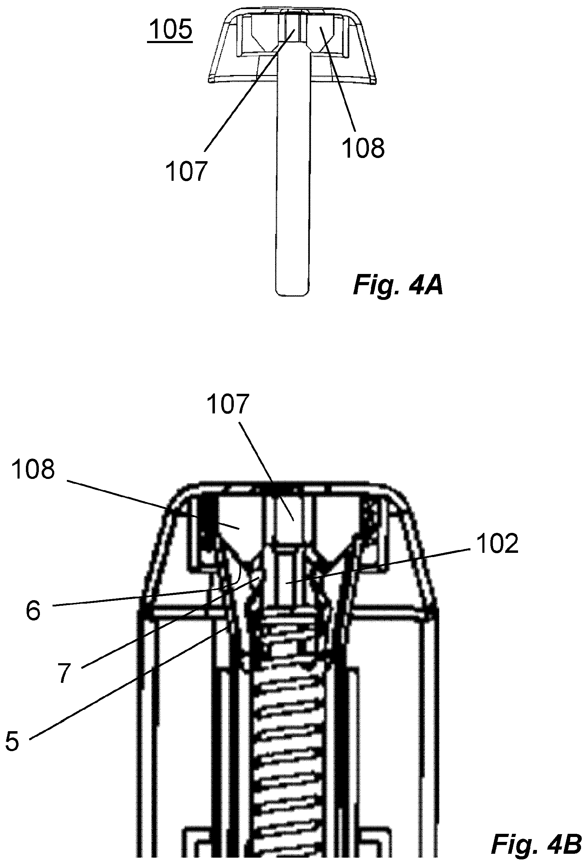

Fig. 4A shows an embodiment of the present disclosure in which the distal end cap 105 comprises a guide hole 107 and a displacement structure 108. As shown in Fig. 4B, the guide hole 107 is configured to axially guide the distal end of the plunger rod 102 when the activation member 1 is distally displaced. In this position, the displacement structure 108 interacts with the inclined end surface 6 of the flexible arms 5 of the activation member 1 and thereby radially outwardly deflects the flexible arms 5, such that the distal end of the plunger rod 102 is released from the plunger rod support 7. According to an embodiment, the displacement structure 108 is radially inclined with the same angle of inclination as the inclined end surface 6 of the flexible arms 5.

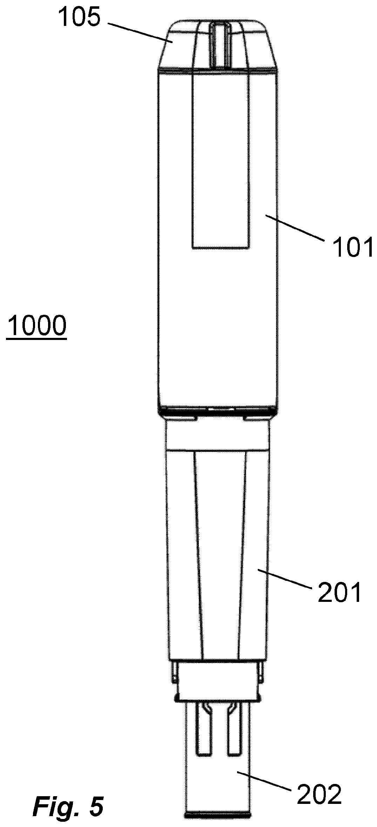

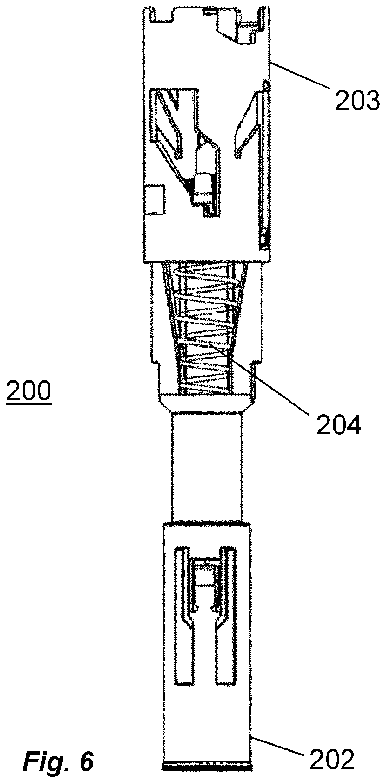

The medicament delivery device 1000 according to a preferred embodiment of the present disclosure is shown in Fig. 5 and comprises a distal subassembly 100 and a proximal subassembly 200 (see Fig. 6). The distal subassembly 100 is a subassembly 100 according to one of the embodiments described in the foregoing paragraphs.

According to an embodiment, the proximal subassembly 200 comprises a proximal housing 201 connectable to the distal housing 101 of the distal subassembly 100 and a displaceable needle cover 202 partially arranged within the proximal housing 201.

As further shown in Fig. 6, according to an embodiment, the proximal subassembly 200 further comprises a rotator 203 coaxially connected to the distal inner part of the proximal housing 201 (see Fig. 5). The rotator 203 is movable in relation to the proximal housing 201. According to an embodiment, the proximal subassembly 200 further comprises a medicament container holder (not visible in Figs. 5 and 6) connected through the needle cover 202 and the rotator 203 to the proximal housing 201.

According to an embodiment, the proximal subassembly 200 further comprises a needle cover spring 204 disposed between the needle cover 202 and the medicament container holder and configured to apply a biasing force on the needle cover 202. The needle cover 202 is axially movable inside at least the proximal housing 201 such that the distal end of the needle cover 202 contacts the contact surface 4 of the activation member 1, when the needle cover is moved distally in order to distally displace the activation member 1.

While the invention has been illustrated and described in detail in the drawings and the foregoing description, such illustration and description are to be considered illustrative or exemplary and not restrictive. It should be noted that the invention covers all further features shown in the figures individually although they may not have been described in the afore description and it will be understood that changes and modifications may be made by those of ordinary skill within the scope of the following claims. In particular, the present invention covers further embodiments with any combination of features from different embodiments described above and below.

Furthermore, in the claims the word "comprising" does not exclude other elements or steps, and the terms "a", "an" "first", "second" etc. do not exclude a plurality. A single unit may fulfil the functions of several features recited in the claims. The terms "essentially", "about", "approximately" and the like in connection with an attribute or a value particularly also define exactly the attribute or exactly the value, respectively. Any reference signs in the claims should not be construed as limiting the scope.

## Claims

1. A medicament delivery device (1000), comprising:
- an activation member (1) that extends along a longitudinal axis from a proximal to a distal end,
- a needle cover (202),
- a plunger rod (102),
- a drive spring (103),
- a distal end cap (105),
- a distal housing (101), wherein the activation member (1) is coaxially arranged within the distal housing (101) and distally movable in relation to the distal housing (101), wherein the distal end cap (105) is connected to the distal housing (101),
- a distal spring (106) arranged between the distal end cap (105) and the activation member (1) configured to apply a biasing force on the activation member (1),
the activation member (1) comprising an outer part (2) and an inner part (3) coaxially connected to the outer part (2), wherein the outer part (2) comprises a needle cover contact surface (4) configured to contact the needle cover (202); wherein the inner part (3) comprises at least one flexible arm (5) extending distally and having an inclined end surface (6) and a plunger rod support (7) at its distal end, the inclined end surface (6) being in proximal direction inclined in a radial inward direction;
**characterized in that**
the activation member (1) comprises at least two spring supports (8) each extending distally and being configured to support a distal end of the drive spring (103), wherein the drive spring (103) is coaxially arranged between the plunger rod (102) and the spring supports (8) of the activation member (1), a distal end of the drive spring (103) being supported by the spring supports (8) and a proximal end of the drive spring (103) being supported by a proximal ledge (104) of the plunger rod (102);
the plunger rod support (7) is configured to engage with the plunger rod (102) in order to inhibit a proximal movement of the plunger rod (102);
the inclined end surface (6) is configured to interact with the distal end cap (105), causing a radial outward deflection of the at least one flexible arm (5) thereby disengaging the plunger rod support (7) from the plunger rod (102), when the activation member (1) is distally displaced by the needle cover (202);
the plunger rod (102) is coaxially arranged within the activation member (1) and releasably connected to the plunger rod support (7) of the activation member (1);
the distal end cap (105) comprises a displacement structure (108) configured to interact with the inclined end surface (6) of the at least one flexible arm (5) in order to radially deflect the at least one flexible arm (5) such that the plunger rod support (7) is disengaged from the plunger rod (102), when the activation member (1) is distally displaced;
the displacement structure (108) is rigid and is radially inclined, preferably with the same angle of inclination as the inclined end surface (6) of the at least one flexible arm (5).

2. The medicament delivery device (1000) of claim 1, wherein a circumferential surface of the inner part (3) is formed of the at least one flexible arm (5), the at least two spring supports (8), and at least one slot between each flexible arm (5) and each spring support (8), wherein the at least one slot is configured to kinematically decouple the deflection of each flexible arm (5) from the spring supports (8).

3. The medicament delivery device (1000) of claim 1 or 2, wherein the inner part (3) and the outer part (2) overlap along a part of their length.

4. The medicament delivery device (1000) of any one of the preceding claims, wherein the outer part (2) is connected to or integral with the inner part (3) via at least one support (9) extending radially between at least one of the spring supports (8) and a connection structure (10) of the outer part (2).

5. The activation member (1) of any one of the preceding claims, wherein each plunger rod support (7) has a radially inwards extending protrusion configured to form a stop surface for engagement with a distal portion of the plunger rod (102).

6. The medicament delivery device (1000) of any one of the preceding claims, wherein each spring support (8) comprises a protrusion or step extending radially inwards and having a proximally facing support surface (11) configured to support the distal end of the drive spring (103).

7. The medicament delivery device (1000) of any one of the preceding claims, further comprising a distal spring support surface (12) configured to interact with a distal spring (106) of the medicament delivery device (1000), wherein the distal spring support surface (12) is formed by the support (9) or a distal end surface of the connection structure (10).

8. The medicament delivery device (1000) of any one of the preceding claims, wherein the at least one flexible arm (5) and the at least two spring supports (8) are alternately arranged in circumferential direction.

9. The medicament delivery device (1000) of any one of claims 6 to 8, wherein the proximally facing support surface (11) of the protrusion or step of the spring support (8) is located more proximal compared to the distally facing stop surface of the radially inwards extending protrusion of the plunger rod support (7).

10. The medicament delivery device (1000) of any of the previous claims, wherein the distal end cap (105) comprises a guide hole along the longitudinal axis of the medicament delivery device (1000) configured to axially guide the distal end of the plunger rod (102) when the activation member (1) is distally displaced.

11. The medicament delivery device (1000) of any of the previous claims, comprising:
- a proximal housing (201) connectable to the distal housing (101);
- a rotator (203) coaxially connected to a distal inner part of the proximal housing (201) and movable in relation to the proximal housing (201);
- a displaceable needle cover (202) partially arranged within the proximal housing (201);
- a medicament container holder connected through the needle cover (202) and the rotator (203) to the proximal housing (201) and configured to receive a medicament container;
- a needle cover spring (204) disposed between the needle cover (202) and the medicament container holder and configured to apply a biasing force on the needle cover (202),
wherein the needle cover (202) is axially movable inside at least the proximal housing (201) such that a distal end of the needle cover (202) contacts the contact surface of the activation member (1) whereby the activation member (1) is distally displaced.

12. The medicament delivery device (1000) of any of the previous claims, wherein the medicament delivery device (1000) is a disposable single-dose auto-injector.

## Patentansprüche

1. Medikamentenabgabevorrichtung (1000), umfassend:
- ein Betätigungselement (1), das sich entlang einer Längsachse von einem proximalen zu einem distalen Ende erstreckt,
- eine Nadelabdeckung (202),
- eine Kolbenstange (102),
- eine Antriebsfeder (103),
- eine distale Endkappe (105),
- ein distales Gehäuse (101), wobei das Aktivierungselement (1) innerhalb des distalen Gehäuses (101) koaxial angeordnet ist und in Bezug auf das distale Gehäuse (101) distal beweglich ist, wobei die distale Endkappe (105) mit dem distalen Gehäuse (101) verbunden ist,
- eine distale Feder (106), die zwischen der distalen Endkappe (105) und dem Aktivierungselement (1) angeordnet und konfiguriert ist, um eine Vorspannkraft auf das Aktivierungselement (1) auszuüben,
das Aktivierungselement (1) umfassend ein äußeres Teil (2) und ein inneres Teil (3), das mit dem äußeren Teil (2) koaxial verbunden ist, wobei das äußere Teil (2) eine Nadelabdeckungs-Kontaktoberfläche (4) umfasst, die konfiguriert ist, um die Nadelabdeckung (202) zu kontaktieren; wobei das innere Teil (3) mindestens einen flexiblen Arm (5) umfasst, der sich distal erstreckt und eine geneigte Endoberfläche (6) und eine Kolbenstangenstütze (7) an seinem distalen Ende aufweist, wobei die geneigte Endoberfläche (6) in proximaler Richtung in einer radialen Einwärtsrichtung geneigt ist;
**dadurch gekennzeichnet, dass**
das Aktivierungselement (1) mindestens zwei Federstützen (8), die sich jeweils distal erstrecken, umfasst und konfiguriert sind, um ein distales Ende der Antriebsfeder (103) zu stützen, wobei die Antriebsfeder (103) zwischen der Kolbenstange (102) und den Federstützen (8) des Aktivierungselements (1) koaxial angeordnet ist, wobei ein distales Ende der Antriebsfeder (103) durch die Federstützen (8) gestützt wird und ein proximales Ende der Antriebsfeder (103) durch einen proximalen Absatz (104) der Kolbenstange (102) gestützt wird;
die Kolbenstangenstütze (7) konfiguriert ist, um mit der Kolbenstange (102) in Eingriff zu treten, um eine proximale Bewegung der Kolbenstange (102) zu verhindern;
die geneigte Endoberfläche (6) konfiguriert ist, um mit der distalen Endkappe (105) zusammenzuwirken, wobei eine radiale Auslenkung nach außen des mindestens einen flexiblen Arms (5) bewirkt wird, wodurch die Kolbenstangenstütze (7) von der Kolbenstange (102) gelöst wird, wenn das Aktivierungselement (1) durch die Nadelabdeckung (202) distal verschoben wird;
die Kolbenstange (102) innerhalb des Aktivierungselements (1) koaxial angeordnet ist und mit der Kolbenstangenstütze (7) des Aktivierungselements (1) lösbar verbunden ist;
die distale Endkappe (105) eine Verschiebungsstruktur (108) umfasst, die konfiguriert ist, um mit der geneigten Endoberfläche (6) des mindestens einen flexiblen Arms (5) zusammenzuwirken, um den mindestens einen flexiblen Arm (5) radial auszulenken, derart, dass die Kolbenstangenstütze (7) von der Kolbenstange (102) gelöst wird, wenn das Aktivierungselement (1) distal verschoben wird;
die Verschiebungsstruktur (108) starr ist und radial geneigt ist, vorzugsweise mit demselben Neigungswinkel wie die geneigte Endoberfläche (6) des mindestens einen flexiblen Arms (5).

2. Medikamentenabgabevorrichtung (1000) nach Anspruch 1, wobei eine umlaufende Oberfläche des inneren Teils (3) aus dem mindestens einen flexiblen Arm (5), den mindestens zwei Federstützen (8) und mindestens einem Schlitz zwischen jedem flexiblen Arm (5) und jeder Federstütze (8) ausgebildet ist, wobei der mindestens eine Schlitz konfiguriert ist, um die Auslenkung jedes flexiblen Arms (5) von den Federstützen (8) kinematisch zu entkoppeln.

3. Medikamentenabgabevorrichtung (1000) nach Anspruch 1 oder 2, wobei das innere Teil (3) und das äußere Teil (2) entlang eines Teils ihrer Länge überlappen.

4. Medikamentenabgabevorrichtung (1000) nach einem der vorstehenden Ansprüche, wobei das äußere Teil (2) mit dem inneren Teil (3) über mindestens eine Stütze (9), die sich radial zwischen mindestens einer der Federstützen (8) und einer Verbindungsstruktur (10) des äußeren Teils (2) erstreckt, verbunden oder aus einem Stück ist.

5. Aktivierungselement (1) nach einem der vorstehenden Ansprüche, wobei jede Kolbenstangenstütze (7) einen sich radial nach innen erstreckenden Vorsprung aufweist, der konfiguriert ist, um eine Anschlagoberfläche für den Eingriff mit einem distalen Abschnitt der Kolbenstange (102) auszubilden.

6. Medikamentenabgabevorrichtung (1000) nach einem der vorstehenden Ansprüche, wobei jede Federstütze (8) einen Vorsprung oder Schritt umfasst, der sich radial nach innen erstreckt und eine nach proximal weisende Stützoberfläche (11) aufweist, die konfiguriert ist, um das distale Ende der Antriebsfeder (103) zu stützen.

7. Medikamentenabgabevorrichtung (1000) nach einem der vorstehenden Ansprüche, ferner umfassend eine distale Federstützoberfläche (12), die konfiguriert ist, um mit einer distalen Feder (106) der Medikamentenabgabevorrichtung (1000) zusammenzuwirken, wobei die distale Federstützoberfläche (12) durch die Stütze (9) oder eine distale Endoberfläche der Verbindungsstruktur (10) ausgebildet ist.

8. Medikamentenabgabevorrichtung (1000) nach einem der vorstehenden Ansprüche, wobei der mindestens eine flexible Arm (5) und die mindestens zwei Federstützen (8) abwechselnd in Umfangsrichtung angeordnet sind.

9. Medikamentenabgabevorrichtung (1000) nach einem der Ansprüche 6 bis 8, wobei sich die nach proximal weisende Stützoberfläche (11) des Vorsprungs oder Schritts der Federstütze (8) weiter proximal befindet als die nach distal weisende Anschlagoberfläche des sich radial nach innen erstreckenden Vorsprungs der Kolbenstangenstütze (7).

10. Medikamentenabgabevorrichtung (1000) nach einem der vorstehenden Ansprüche, wobei die distale Endkappe (105) ein Führungsloch entlang der Längsachse der Medikamentenabgabevorrichtung (1000) umfasst, das konfiguriert ist, um das distale Ende der Kolbenstange (102) axial zu führen, wenn das Aktivierungselement (1) distal verschoben wird.

11. Medikamentenabgabevorrichtung (1000) nach einem der vorstehenden Ansprüche, umfassend:
- ein proximales Gehäuse (201), das mit dem distalen Gehäuse (101) verbindbar ist;
- einen Rotator (203), der mit einem distalen inneren Teil des proximalen Gehäuses (201) koaxial verbunden und in Bezug auf das proximale Gehäuse (201) beweglich ist;
- eine verschiebbare Nadelabdeckung (202), die teilweise innerhalb des proximalen Gehäuses (201) angeordnet ist;
- einen Medikamentenbehälterhalter, der über die Nadelabdeckung (202) und den Rotator (203) mit dem proximalen Gehäuse (201) verbunden und konfiguriert ist, um einen Medikamentenbehälter aufzunehmen;
- eine Nadelabdeckungsfeder (204), die zwischen der Nadelabdeckung (202) und dem Medikamentenbehälterhalter eingerichtet und konfiguriert ist, um eine Vorspannkraft auf die Nadelabdeckung (202) auszuüben,
wobei die Nadelabdeckung (202) im Inneren mindestens des proximalen Gehäuses (201) derart axial beweglich ist, dass ein distales Ende der Nadelabdeckung (202) die Kontaktoberfläche des Aktivierungselements (1) kontaktiert, wodurch das Aktivierungselement (1) distal verschoben wird.

12. Medikamentenabgabevorrichtung (1000) nach einem der vorstehenden Ansprüche, wobei die Medikamentenabgabevorrichtung (1000) ein Einweg-Einzeldosis-Autoinjektor ist.

## Revendications

1. Dispositif d'administration de médicament (1000), comprenant :
- un élément d'activation (1) qui s'étend le long d'un axe longitudinal d'une extrémité proximale à une extrémité distale,
- une protection d'aiguille (202),
- une tige de piston (102),
- un ressort d'entraînement (103),
- un capuchon d'extrémité distale (105),
- un logement distal (101), dans lequel l'élément d'activation (1) est agencé coaxialement au sein du logement distal (101) et mobile de manière distale par rapport au logement distal (101), dans lequel le capuchon d'extrémité distale (105) est relié au logement distal (101),
- un ressort distal (106) agencé entre le capuchon d'extrémité distale (105) et l'élément d'activation (1) conçu pour appliquer une force de sollicitation sur l'élément d'activation (1),
l'élément d'activation (1) comprenant une partie externe (2) et une partie interne (3) reliée coaxialement à la partie externe (2), dans lequel la partie externe (2) comprend une surface de contact de protection d'aiguille (4) conçue pour entrer en contact avec la protection d'aiguille (202) ; dans lequel la partie interne (3) comprend au moins un bras flexible (5) s'étendant de manière distale et ayant une surface d'extrémité inclinée (6) et un support de tige de piston (7) au niveau de son extrémité distale, la surface d'extrémité inclinée (6) étant dans une direction proximale inclinée dans une direction radiale vers l'intérieur ;
**caractérisé en ce que**
l'élément d'activation (1) comprend au moins deux supports de ressort (8) s'étendant chacun de manière distale et étant conçus pour supporter une extrémité distale du ressort d'entraînement (103), dans lequel le ressort d'entraînement (103) est agencé coaxialement entre la tige de piston (102) et les supports de ressort (8) de l'élément d'activation (1), une extrémité distale du ressort d'entraînement (103) étant supportée par les supports de ressort (8) et une extrémité proximale du ressort d'entraînement (103) étant supportée par un rebord proximal (104) de la tige de piston (102) ;
le support de tige de piston (7) est conçu pour venir en prise avec la tige de piston (102) afin d'inhiber un mouvement proximal de la tige de piston (102) ;
la surface d'extrémité inclinée (6) est conçue pour interagir avec le capuchon d'extrémité distale (105), provoquant une déviation radiale vers l'extérieur de l'au moins un bras flexible (5), mettant de ce fait hors de prise le support de tige de piston (7) de la tige de piston (102), lorsque l'élément d'activation (1) est déplacé de manière distale par la protection d'aiguille (202) ;
la tige de piston (102) est agencée coaxialement au sein de l'élément d'activation (1) et reliée de manière amovible au support de tige de piston (7) de l'élément d'activation (1) ;
le capuchon d'extrémité distale (105) comprend une structure de déplacement (108) conçue pour interagir avec la surface d'extrémité inclinée (6) de l'au moins un bras souple (5) afin de dévier radialement l'au moins un bras flexible (5) de telle sorte que le support de tige de piston (7) est mis hors de prise d'avec la tige de piston (102), lorsque l'élément d'activation (1) est déplacé de manière distale ;
la structure de déplacement (108) est rigide et est inclinée radialement, de préférence avec le même angle d'inclinaison que la surface d'extrémité inclinée (6) de l'au moins un bras flexible (5).

2. Dispositif d'administration de médicament (1000) selon la revendication 1, dans lequel une surface circonférentielle de la partie interne (3) est formée de l'au moins un bras flexible (5), des au moins deux supports de ressort (8), et d'au moins une fente entre chaque bras flexible (5) et chaque support de ressort (8), dans lequel l'au moins une fente est conçue pour désaccoupler cinématiquement la déviation de chaque bras flexible (5) des supports de ressort (8).

3. Dispositif d'administration de médicament (1000) selon la revendication 1 ou 2, dans lequel la partie interne (3) et la partie externe (2) se chevauchent le long d'une partie de leur longueur.

4. Dispositif d'administration de médicament (1000) selon l'une quelconque des revendications précédentes, dans lequel la partie externe (2) est reliée avec ou solidaire de la partie interne (3) par l'intermédiaire d'au moins un support (9) s'étendant radialement entre au moins l'un des supports de ressort (8) et une structure de liaison (10) de la partie externe (2).

5. Élément d'activation (1) selon l'une quelconque des revendications précédentes, dans lequel chaque support de tige de piston (7) a une saillie s'étendant radialement vers l'intérieur conçue pour former une surface d'arrêt pour une mise en prise avec une partie distale de la tige de piston (102).

6. Dispositif d'administration de médicament (1000) selon l'une quelconque des revendications précédentes, dans lequel chaque support de ressort (8) comprend une saillie ou une marche s'étendant radialement vers l'intérieur et ayant une surface de support (11) orientée de manière proximale conçue pour supporter l'extrémité distale du ressort d'entraînement (103).

7. Dispositif d'administration de médicament (1000) selon l'une quelconque des revendications précédentes, comprenant en outre une surface de support de ressort distal (12) conçue pour interagir avec un ressort distal (106) du dispositif d'administration de médicament (1000), dans lequel la surface de support de ressort distal (12) est formée par le support (9) ou une surface d'extrémité distale de la structure de liaison (10).

8. Dispositif d'administration de médicament (1000) selon l'une quelconque des revendications précédentes, dans lequel l'au moins un bras flexible (5) et les au moins deux supports de ressort (8) sont agencés en alternance dans une direction circonférentielle.

9. Dispositif d'administration de médicament (1000) selon l'une quelconque des revendications 6 à 8, dans lequel la surface de support (11) orientée de manière proximale de la saillie ou de la marche du support de ressort (8) est située plus proximale par comparaison avec la surface d'arrêt orientée de manière distale de la saillie s'étendant radialement vers l'intérieur du support de tige de piston (7).

10. Dispositif d'administration de médicament (1000) selon l'une quelconque des revendications précédentes, dans lequel le capuchon d'extrémité distale (105) comprend un trou de guidage le long de l'axe longitudinal du dispositif d'administration de médicament (1000) conçu pour guider axialement l'extrémité distale de la tige de piston (102) lorsque l'élément d'activation (1) est déplacé de manière distale.

11. Dispositif d'administration de médicament (1000) selon l'une quelconque des revendications précédentes, comprenant :
- un logement proximal (201) pouvant être relié au logement distal (101) ;
- un rotateur (203) relié de manière coaxiale à une partie interne distale du logement proximal (201) et mobile par rapport au logement proximal (201) ;
- une protection d'aiguille (202) déplaçable agencée partiellement au sein du logement proximal (201) ;
- un élément de retenue de récipient de médicament relié par le biais de la protection d'aiguille (202) et du rotateur (203) au logement proximal (201) et conçu pour recevoir un récipient de médicament ;
- un ressort de protection d'aiguille (204) disposé entre la protection d'aiguille (202) et l'élément de retenue de récipient de médicament et conçu pour appliquer une force de sollicitation sur la protection d'aiguille (202),
dans lequel la protection d'aiguille (202) peut être entraînée en mouvement axialement à l'intérieur d'au moins le logement proximal (201) de telle sorte qu'une extrémité distale de la protection d'aiguille (202) entre en contact avec la surface de contact de l'élément d'activation (1), moyennant quoi l'élément d'activation (1) est déplacé de manière distale.

12. Dispositif d'administration de médicament (1000) selon l'une quelconque des revendications précédentes, dans lequel le dispositif d'administration de médicament (1000) est un auto-injecteur monodose jetable.
